# EUROPEAN PATENT APPLICATION

(11) **EP 4 124 290 A1**
(43) Date of publication of application: **01.02.2023**
(21) Application number: 21187861.6
(22) Date of filing: 27.07.2021
(51) Int. Cl.: A61B 5/0531, A61B 5/00, A61B 5/0533

(54) **PAIN AND/OR NON-PAIN AROUSAL DETECTION DURING ORAL CARE**

(71) Applicant: Koninklijke Philips N.V., 5656 AG Eindhoven (NL)
(72) Inventor: SPELT, Hanne Adriana Alijda, 5656 AE Eindhoven (NL); GERHARDT, Lutz Christian, 5656 AE Eindhoven (NL); JOHNSON, Mark Thomas, 5656 AE Eindhoven (NL); HIWALE, Sujitkumar, 5656 AE Eindhoven (NL)
(74) Representative: Philips Intellectual Property & Standards

(57) **Abstract**

A processor and method for detecting pain and non-pain arousal responsive to physical stimuli applied to oral surfaces of a subject. A biological signal such as skin conductance, or another physiological parameter, can be used as a proxy measure for acute pain and/or non-pain arousal. The instances of mechanical engagement with the oral surfaces can be detected or tracked. Differential detection of pain vs non-pain arousal is achieved based on assessing signal synchronization or correlation between the biological signal and the oral contact events.

## Description

### FIELD OF THE INVENTION

This invention relates to means for monitoring a subject's pain and/or non-pain biological arousal during an oral care session.

### BACKGROUND OF THE INVENTION

Patients can experience pain during dental examination or treatment, for example during such interventions as pocket probing, drilling or tightening of braces. For example, it has been found that 90% of patients experience pain during scaling and root planing. It is important, both for the patient and dental practitioner, to have an accurate estimate of the patient's pain experience, to enable improvement in the dental intervention or procedure. Pain can guide the practitioner during treatment and optimize workflow. Pain also plays a major role in the onset of dental anxiety and stress. Therefore, an objective real-time measurement of pain would be beneficial.

However, it is difficult to determine the level of pain. A common method for measuring the level of pain is for example the Visual Analogue Scale (VAS), in relation to which reference is made to the paper: Huskisson, Edward C. "Measurement of pain.", The lancet, 304.7889 (1974): 1127-1131.

This method is however highly subjective. It is also non-automatized. Furthermore, the scale is typically employed only once, after dental treatment has finished, and thus this method of pain measurement is useful only for measuring relative changes in the pain of a given patient over multiple different sessions. To better guide the dental practitioner, a more objective, and preferably more sensitive and accurate, pain assessment would be of value. Preferably a method with greater temporal resolution would also be valuable so that pain level can be assessed at a series of moments during a procedure, rather than just at the end.

It is known that certain physiological signals can change in dependence upon a patient subjective/psychological response to a physical stimulus (e.g. pain or other negatively valenced emotion). One such physiological signal is skin conductance which can be measured using galvanic skin resistance. Skin conductance measurements reflect changes in resistance and electrical conductivity in the skin as result of physiological arousal that can indicate both acute pain and also psychological stress. Another example is heart rate or heart rate variability. Physiological measures provide a more objective quantification of pain and other arousal responses. In addition, they are able to be measured automatically and continuously, have a high temporal resolution (enabling sensitive assessment), and can provide real-time assessment.

However, it is difficult to distinguish in the physiological signal whether a subject is experiencing acute pain, or whether they are merely experiencing another non-pain arousal response, such as stress. Both lead to physiological arousal and thus a change of relevant biological signals. Therefore, it would be of advantage to provide a means for using biological signals to monitor a patient in a way that can differentially distinguish pain from non-pain arousal states.

### SUMMARY OF THE INVENTION

The invention is defined by the claims.

According to examples in accordance with an aspect of the invention, there is provided a processor adapted for classifying a subject's response to a series of contact events during a measurement session, the processor adapted to:
receive, during the measurement session, an indication of timings of a series of oral contact events, each contact event corresponding to mechanical engagement of an oral tool with a surface within the oral cavity of a subject;
receive, during the measurement session, a biological signal for the subject from a biological signal sensing apparatus coupled to the subject;
identify physiological response events in the biological signal;
determine a temporal proximity between each physiological response event and an immediately preceding contact event, and classify a subject pain and/or non-pain arousal state associated with the physiological response event based on the temporal proximity.

The problem to be solved is to enable real-time or post-hoc determination of the extent to which a subject is experiencing pain in response to a force applied to an oral surface (e.g. tooth or gum), or the extent to which they are experiencing non-pain arousal, typically related to a negatively valenced emotion. Physiological parameters such as skin conductance, heart rate and others are known to be correlated with arousal and with pain. A spike in such signals (up or down) closely temporally correlated with occurrence of a contact event is an indication of a pain response (i.e. the physiological response has a causal relationship with the contact, so it is likely to be pain). On the other hand, a spike in the biological signal not closely temporally correlated with a contact event is more likely to be a generalized sympathetic response of the subject to the overall experience, e.g. a psychological stress response. By measuring the temporal proximity between a physiological response event and a preceding contact event, the class of arousal response of the subject can be determined.

The underlying concept therefore is the assessment of temporal correlation between the mechanical engagement with the tooth or oral tissue and the biological signal. The degree of correlation is used to differentially classify a subject response as pain or non-pain arousal. For this purpose, an indication of timings of a series of oral contact events is received, and a temporal relationship between each detected physiological response event is temporally related to the contact events to determine if a causal relationship exists.

The term 'immediately preceding' in the above context means the temporally closest contact event in advance of the physiological response event.

The indication of the timings of the contact events may comprise one or more contact signals received from a contact identification means. In some embodiments, obtaining the indication of the timings of the contact events comprises receiving one or more contact sensing signals from a contact sensing means adapted for detecting instances of mechanical engagement of the oral tool with an oral surface. In other words, in these embodiments, the contact is automatically sensed. In alternative embodiments, a dental practitioner might simply press a button upon each contact event, and wherein this triggers transmission of a contact signal to the processor.

The contact signal may be a discrete signal transmitted to the processor upon each detected contact event. Therefore a plurality of contact signals would typically be received over the measurement session, each reflective of a single contact event. Alternatively, a continuous signal may be coupled from a contact sensing means to the processor and wherein the contact events are identified or extracted from the continuous signal, e.g. as peaks in the signal.

In further examples, the indication of the timings of the contact events (the mechanical stimuli) might be obtained from a set of prompts issued to a user interface prompting a user to apply a stimulus responsive to generation of the prompt (where the prompts may be visual indications on a screen or audible sounds, or haptic signals issued through the tool).

Each contact event may have an event occurrence time and an event duration, and wherein the event duration for each contact event is shorter than the time interval between any two successive contact events. This allows for the interval between two contact events to be long enough to detect the physiological response.

In some embodiments, the biological signal may be a skin conductance signal.

In some embodiments, each physiological response event may be characterized by an event fingerprint in the biological signal comprising a spike up or down in the signal, for example a peak or drop in the signal having a peak or drop height exceeding a pre-defined threshold, e.g. from a baseline.

A physiological event is detected by detecting a pre-defined signature or fingerprint in the biological signal. This typically comprises a spike in the signal, in particular relative to a baseline of the signal, where the baseline may be the average trend line of the signal, or may be a fixed offset (e.g. determined based on an initial baseline measurement of the biological signal before the measurement session begins). The baseline may be recurrently re-calculated throughout the measurement session.

In some cases, the event fingerprint may also have a threshold time duration and/or require a threshold upward gradient of the leading/rising edge of the peak.

In some embodiments, the processor may be configured to classify a physiological response event as associated with a pain state dependent on the physiological response event occurring within a pre-defined time window immediately following a contact event. In this case it can be assumed that the elevated biological signal reading has been caused by the contact event, and therefore that it is a pain response, rather than a generalized psychological stress response.

The time window may be measured from the first onset of the contact event. The occurrence time of the physiological response event may be taken to be a first onset time of the physiological response.

The classification may comprise determining a pain level of the pain state based on one or more properties of the physiological response event peak/drop, and preferably wherein the one or more properties include a maximum height of a leading edge of the peak/drop. The one or more properties may include a time duration spanned by the event peak/drop, or an area under the peak/drop relative to a baseline, and/or may include a frequency-domain analysis (e.g. a particular spectral signature or particular spectral components of the peak/drop).

In some embodiments, the processor may be configured to classify a physiological response event as associated with a non-pain arousal state dependent on the physiological response event occurring outside of a time window of pre-defined duration following any contact event.

In some embodiments, the classification may be performed in real time during the measurement session. A user interface may be connected with the system. The processor in some embodiments may be adapted to generate real-time feedback during the measurement session indicative of the classifications, and to communicate the feedback to a user interface for presentation to a user.

In some embodiments, the processor may be adapted to compute a total pain amount for the measurement session, the total pain amount being a product of a pain level and a time for which the pain was experienced, the pain level being computed for each physiological response event peak having a pain classification based on one or more properties of the physiological response event peak/drop.

Additionally or alternatively, the processor may be adapted to compute a total non-pain arousal amount over the time period, the total non-pain arousal amount being a product of an arousal level and a time for which the arousal was experienced, the arousal level being computed for each physiological response event peak having a non-pain arousal classification based on one or more properties of the physiological response event peak/drop.

The processor may be adapted to generate a data signal or packet representative of the computed total pain amount and/or total non-pain arousal amount, which may be output from the processor, e.g. to a memory or to another device. The processor may further be adapted to generate user feedback indicative of the computed total pain amount and/or total non-pain arousal amount.

In some embodiments, the processor may be adapted to trigger one or more response actions dependent on the total pain amount and/or total non-pain arousal amount.

In cases where the system is configured for home use, the response actions could include one or more of: adjusting settings of the tool, for example changing a toothbrush drive setting to a more gentle brushing setting; transmitting a feedback message to a dental practitioner; and scheduling an appointment with a dental practitioner at a future time dependent on the pain level.

A further aspect of the invention provides a system. The system comprises a contact identification means for identifying timings of a series of oral contact events, each contact event corresponding to mechanical engagement of an oral tool with a surface within the oral cavity of a subject. The system further comprises a biological signal sensing apparatus for measuring a physiological response of the subject. The system further comprises a processor in accordance with any example or embodiment outlined above or described below, or in accordance with any claim of this application. The processor is arranged to receive, over a measurement session, one or more contact signals from the contact identification means indicative of timings of the series of oral contact events and a biological signal from the biological signal sensing apparatus.

In some embodiments, the system may include the oral tool. The contact identification means may be a contact sensing means. It may comprise a contact sensor integrated in the oral tool, for detecting contact between a portion of the tool and an oral surface.

In some alternative embodiments, the contact identification means may detect engagement of a tooth by a fluid emission of the tool, e.g. a liquid or air jet.

The contact identification means in some embodiments may be separate to the tool itself, e.g. a remote tracking device using optical or electromagnetic means.

In some embodiments, the oral tool may be a toothbrush and the contact identification means may be a sensing means adapted to detect physical contact of an element of a brush head of the toothbrush with an oral surface.

Alternatively, the tool may be a brushing mouthpiece device or an oral irrigator, or a powered flosser. Alternatively, the tool may be a dental probe.

The contact identification means may in some embodiments be a sensing means and may include one or more of: a pressure sensor, a capacitive contact sensor, an optical contact sensor, an inductive contact sensor, an electromagnetic-based contact sensor, or any other type of contact sensor.

In some embodiments, the system may comprise the tool, and wherein the tool comprises an actuable tooth engagement mechanism, adapted when actuated to apply a standardized mechanical stimulus to an oral surface, and wherein the contact signal(s) are obtained based on reading an actuation drive signal of the tooth engagement mechanism.

For example, where the tool is a powered toothbrush, the tooth engagement mechanism might comprise driving a controlled tapping action by the protruding cleaning elements of the toothbrush head. This might be a cyclical tapping motion in some examples.

For example, in one set of embodiments, the tool may be a powered toothbrush having a motor arranged to drive cleaning elements of a brush head in an oscillatory motion pattern, and wherein the oscillatory motion pattern provides the mechanical engagement of the tool with the oral surface, and wherein the contact identification means is duty cycle timer associated with the motor.

Where the tool is an oral irrigator or powered flosser, the tooth engagement mechanism might comprise emitting a controlled pulse of fluid from a fluid jet.

A further aspect of the invention provides a computer-implemented method comprising:
receiving, during the measurement session, an indication of timings of a series of oral contact events, each contact event corresponding to mechanical engagement of an oral tool with a surface within the oral cavity of a subject;
receiving, during the measurement session, a biological signal for the subject from a biological signal sensing apparatus coupled to the subject;
identifying physiological response events in the biological signal;
determining a temporal proximity between each physiological response event and an immediately preceding contact event, and classifying a subject pain and/or non-pain arousal state associated with the physiological response event based thereon.

The indication of the timings may comprise one or more contact signals received from a contact identification means. The contact identification means may be a contact sensing means.

A further aspect of the invention provides a computer program product comprising code means configured to cause a processor to perform a method in accordance with any example or embodiment outlined above or described below, or in accordance with any claim of this application, when the processor is operatively coupled with: a contact identification means adapted for identifying timings of physical engagement of an oral tool with a surface within the oral cavity of a subject; and a biological signal sensing apparatus coupled to the subject.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an example processor in accordance with one or more embodiments;
Figs. 2-4 illustrate analysis of temporal proximity (or temporal correlation) between contact events and physiological response events;
Fig. 5 illustrates an example processing workflow in accordance with one or more embodiments;
Fig. 6 illustrates an example home-use oral care device having integrated means for performing a method in accordance with the present invention;
Fig. 7 illustrates a circuit diagram of the oral care device of Fig. 6; and
Fig. 8 shows an example system for clinical use by a dental practitioner, and which is an embodiment of the present invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a processor and method for detecting pain and non-pain arousal responsive to physical stimuli applied to oral surfaces of a subject. A biological signal such as skin conductance, or another physiological parameter, can be used as a proxy measure for acute pain and/or non-pain arousal. The instances of mechanical engagement with the oral surfaces can be detected or tracked. Differential detection of pain vs non-pain arousal is achieved based on assessing signal synchronization or correlation between the biological signal and the oral contact events Close temporal correlation between a contact event and a physiological response event may indicate the response event is directly causally related to the contact, and thus is a pain response. Absence of close temporal correlation may indicate that the response is more generalized, and thus more likely to be indicative of non-pain arousal, such as psychological stress.

In one set of embodiments, skin conductance measurement is used as the biological signal. A brief introduction to skin conductance measurement will now be outlined.

Skin conductance is mediated through electrodermal activity, i.e. sweating. This has been shown to be linked to pain experiences (see e.g. H. Storm, "Changes in skin conductance as a tool to monitor nociceptive stimulation and pain," Curr. Opin. Anaesthesiol., vol. 21, no. 6, pp. 796-804, 2008.)

The electrodermal system behaves uniformly across the body. However, the eccrine palmar and plantar sweat glands are especially responsive to psychological stimuli, such as pain. High skin conductance levels and peaks in the skin conductance signal, reflect emotional arousal, whereas low levels and smooth signals reflect relaxation and comfort (see e.g. S. D. Kreibig, "Autonomic nervous system activity in emotion: A review," Biol. Psychol., vol. 84, no. 3, pp. 394-421, 2010.).

In painful situations, the electrodermal system becomes activated and the sweat glands fill up. This causes a reduction in skin resistance, and skin conductance consequently increases. Shortly after, the values return to their previous (baseline) levels. This produces an identifiable peak in the skin conductance signal (which may be referred to as an electrodermal response, EDR), and the amplitude of the peak illustrates the firing strength of the sympathetic nerve, which is in turn correlated with acute pain level. These peaks are stimulus-specific, appear after a short time delay (typically around 1-2s), and fall off after the event.

Skin conductance can be measured via galvanic skin response (GSR) measurement equipment.

Skin conductance can show both psychological stress and pain. Pain and non-pain arousal events can be detected by analyzing the skin conductance signal, and identifying relevant peaks in the signal. A relevant peak might be identified for example based on the sum of the height of the leading edge in the peak, or the area under the peak curve during a certain time interval, or based on a frequency domain analysis (e.g. based on the peak exhibiting a pre-determined spectral signature or characteristics) Further metrics of the phasic and tonic components of skin conductance can be found in: J. T. Cacioppo, L. G. Tassinary, and G. G. Berntson, The handbook of psychophysiology, 3rd ed., vol. 44. New York: Cambridge University Press, 2007; and also in W. Boucsein, Electrodermal activity, 2nd ed. New York, NY: Springer Science+Business Media, 2012.

Skin conductance is not the only biological signal which is correlated with pain and stress. For example, heart rate, PPG signal, eye movement, facial expressions, or pupil dilation may also be correlated with emotional arousal. For example, for heart rate, a data series of heart rate values as a function of time may be measured, and wherein peaks in the heart rate are used in place of peaks in skin conductance for detecting pain and/or arousal events. In addition, there is a reduction of heart rate variation with pain/non-pain arousal. Therefore one or both of both heart rate and/or heart rate variation (HRV) may be used as the biological/physiological signal in some embodiments. For heart rate, pain causes an upward spike (an elevated peak in the heart rate signal). For HRV, pain causes a downward spike (drop) in the HRV signal.

Another example is detection of facial expressions, which correlate with arousal, e.g. grimace, clench etc. A camera could be used to acquire image data of the face, and a facial analysis algorithm used to detect variations in facial expression.

Another example is measurement of electrical activity in different groups of muscles.

Another example is tracking of pupil size or movement of eyelids, e.g. by means of a camera.

Another example is measurement of blood flow, where blood flow increases responsive to arousal.

Another example is measurement of blood pressure, where blood pressure increases responsive to arousal.

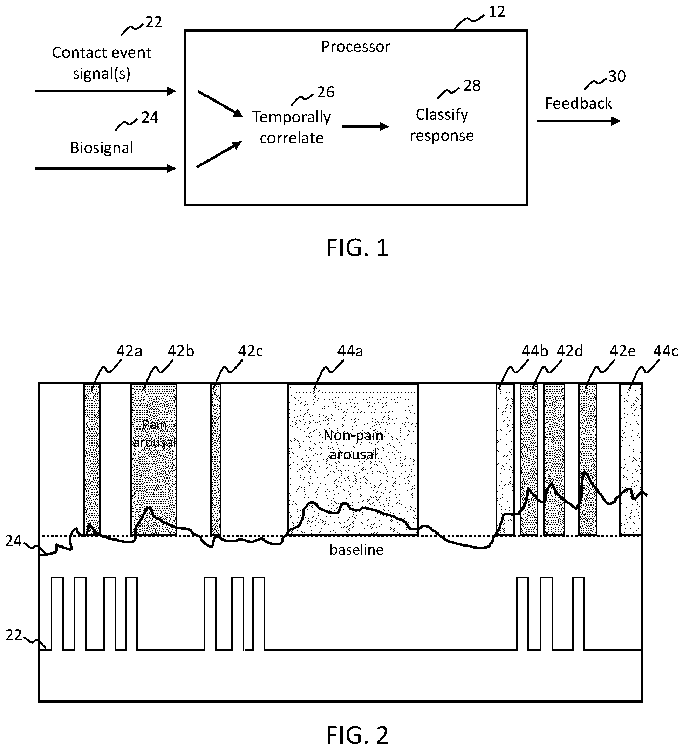

Fig. 1 schematically illustrates the signal inputs, the processing, and the outputs of a processor in accordance with one or more embodiments. The processor is adapted for classifying a subject's response to a series of contact events during a measurement session.

The processor 12 is adapted to receive, during the measurement session, one or more contact signals 22 indicative of timings of a series of oral contact events, each contact event corresponding to mechanical engagement of an oral tool with a surface within the oral cavity of a subject. More generally, the processor might instead receive any signal or data indication of the timings of the contact events, for example one or more data packets carrying the information.

The processor is further adapted to receive, during the measurement session, a biological signal 24 for the subject from a biological signal sensing apparatus coupled to the subject.

The processor is further adapted to identify physiological response events in the biological signal.

The processor is further adapted to determine a temporal proximity 26 between each physiological response event and an immediately preceding contact event. Based on this, the processor is adapted to classify 28 a subject pain and/or non-pain arousal state associated with the physiological response event. The classification may for example be done using a pre-determined algorithm or function, which may be referred to as a classifier algorithm.

A data signal or data packet representative of the derived classification may be generated and provided as a feedback output 30 from the processor. This may be coupled for example to a user interface for communication to a user through a sensory output means of the user interface.

Although Fig. 1 shows a single processor, the steps carried out thereby may be implemented by a processing arrangement comprising multiple processors, or may be implemented by a distributed processing system, e.g. a cloud based processing system. The processor may be operatively coupled to an input/output (I/O) via which the input signals are received and the data outputs are exported.

Descriptions of various embodiments herein refer to determining a temporal proximity between each contact event and a subsequent physiological response event. This will now be described further. The following explanations refer to skin conductance or electrodermal activity in particular. However, it is to be understood that the same principles apply also for other biological signals discussed earlier, and can be applied also for these biological signals without loss of functionality.

As noted above, pain events and non-pain arousal events can be distinguished by checking, for each detected response event in the biological signal, whether that response is correlated or synchronous with a preceding oral contact event. For example, a condition for synchronicity may be pre-defined, which may be based on a pre-defined time window. For example, if a physiological response event falls within a pre-defined time window following a contact event, the physiological response may be classified as a pain response, and if it falls outside of any said time window following any contact event, it may be classified as a non-pain arousal event (e.g. a psychological stress response). The time window may be approximately 2 seconds, for example, between 1 and 2 seconds in some examples. The length of the time window may however be configured as desired, to optimize sensitivity of the pain detection vs accuracy of pain detection. Also, in some scenarios, the time correlation may be slightly offset, leading to an extended delay between the stimulus and the physiological response (e.g. 1-3 seconds, or 0-3 seconds) if the contact stimulus shows an associated latency time. An example of such a latency time is if the physical stimulus comprises a temperature difference from the skin, whereby the heat capacity of the skin causes a latency before the pain is felt.

Fig. 2 schematically illustrates classification of physiological response events based on temporal proximity thereof to preceding contact events. A biological signal 24 as a function of time (x-axis) is shown along with a contact signal 22 as a function of time. The two signals are in temporal registration with one another. In this example, the contact signal is a binary signal, having a value of 1 when contact is occurring and a value of zero when no contact is occurring. This leads to a series of square wave features in the signal, each representing a contact event. The biological signal is a multivalued signal. The biological signal exhibits a plurality of physiological response events, where a physiological response event is characterized by a pre-determined signature or fingerprint in the signal having one or more characteristics. Most typically, a physiological response event may be identified as present between two time points of the biological signal where, between those two time points, the signal is above a pre-defined threshold value/height relative to a baseline level. Additionally or alternatively, there may be other conditions imposed on detection of a physiological response event, where these may include: a peak in the signal of a pre-determined peak height relative to a baseline level; a peak of the signal exhibiting a pre-determined gradient on the leading edge of the peak; a peak of the signal having a pre-defined minimum area under the peak; and/or a peak of the signal exhibiting a pre-defined minimum sum along the leading edge of the peak.

Fig. 2 shows the set of identified physiological response events 42, 44 in the biological signal, where some 42a-42e are characterized as pain response events, and some 44a-44c are characterized as non-pain arousal response events (e.g. psychological stress events).

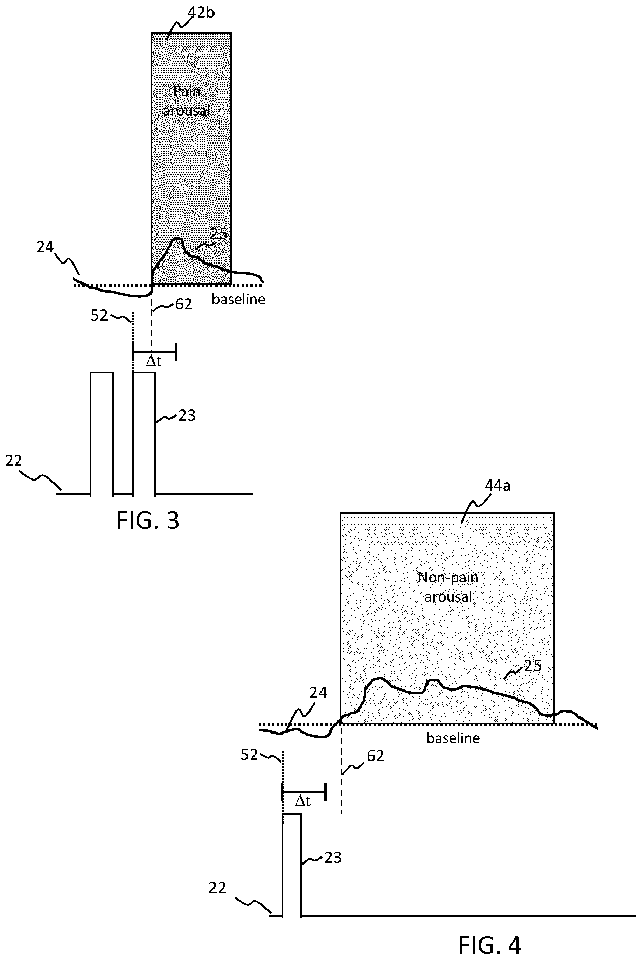

Fig. 3 schematically shows in more detail classification of one of the pain response events 42b of the data series of Fig. 2. The portion of the biological signal 24 and contact signal 22 immediately preceding and throughout the pain event are shown. A physiological response event 25 is identifiable in the biological signal 24 as a peak in the signal, which exceeds a pre-determined peak height. The onset time 62 of the physiological response event peak falls within a predetermined time window, Δt, extending from the onset time 52 of a preceding contact event 23. The physiological response event is therefore classified in this case as a pain event.

Fig. 4 schematically shows in more detail classification of one of the non-pain response events 44a of the data series of Fig. 2. The portion of the biological signal 24 and contact signal 22 immediately preceding and throughout the pain event are shown. A physiological response event 25 is identifiable in the biological signal 24 as a peak in the signal, which exceeds a pre-determined peak height. The onset time 62 of the physiological response event peak falls in this case outside of a predetermined time window, Δt, extending from the onset time 52 of any preceding contact event. In particular, it is outside of the time window, Δt, following the temporally closest contact event 23 preceding onset of the physiological response event. The physiological response event is therefore classified in this case as a non-pain arousal response, e.g. a psychological stress response.

The method employed in embodiments of the present invention may make use of components of a system which comprises one or more of the following components:
a contact identification means for identifying timings of a series of oral contact events, each contact event corresponding to mechanical engagement of an oral tool with a surface within the oral cavity of a subject;
a biological signal sensing apparatus for measuring a physiological response of the subject; and
a processor in accordance with any example or embodiment outlined above or described below, or in accordance with any claim of this application.

By way of one example, the biological signal sensing apparatus may comprise a Galvanic Skin Response (GSR) sensor electrode configuration. The electrodes may be provided in a handheld device, or may form a body-worn apparatus. This may be used to measure the presence and number of electrodermal response events (EDRs) during measurement session, the EDR events corresponding to the physiological response events discussed previously.

The contact identification means may comprise a contact sensor adapted for detecting instances of mechanical engagement of the oral tool with an oral surface, i.e. contact is automatically sensed. Alternatively, a dental practitioner might simply press a button upon each contact event. A discrete signal may be transmitted to the processor upon each detected contact event. Alternatively, a continuous signal may be coupled from the contact sensing means to the processor and wherein the contact events are identified/extract from the continuous signal, e.g. as peaks in the signal.

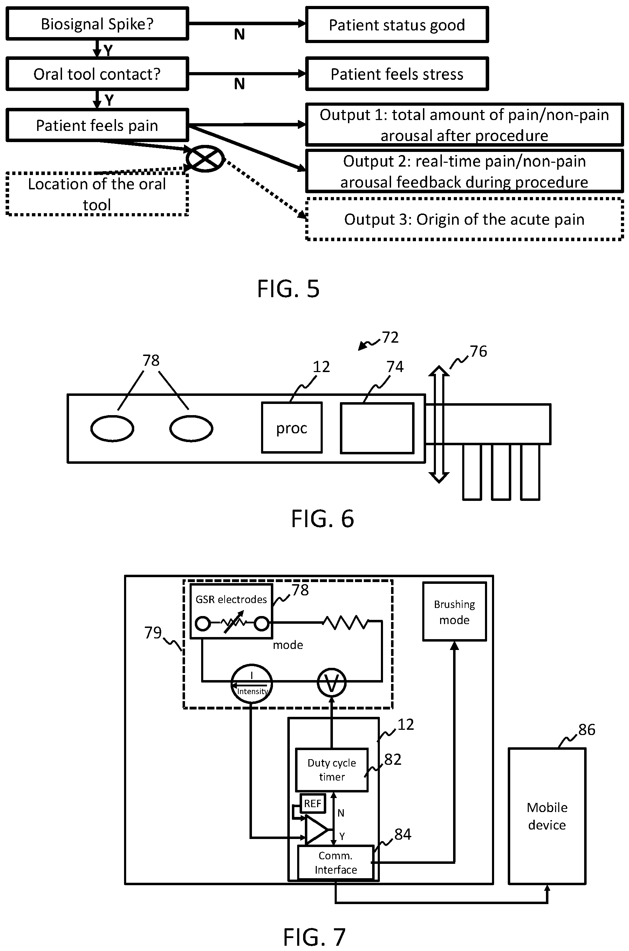

Fig. 5 schematically depicts an example processing workflow which may be employed in embodiments of the present invention.

The processing workflow may comprise a loop in which the biological signal is sampled to detect if a spike is present in the signal corresponding to a physiological response event. If not, then a patient status may be taken to be good. If a physiological response event is detected, the processor next checks the oral contact signal to determine whether the detected physiological response event has occurred within a pre-defined time window following onset of a preceding contact event, e.g. within 0-2 seconds following contact. If so, then the physiological response event may be characterized as a pain event. If not, then the physiological response event may be characterized as a non-pain arousal event, e.g. psychological stress.

The processor may be adapted to compute one or more further parameters related to the physiological response events. For example, the processor may be adapted to compute a pain level of each pain event or a stress level of each stress event. A pain level of the pain state or stress level of the stress state may be computed based on one or more properties of the physiological response event peak or drop, and preferably wherein the one or more properties include a maximum height of a leading edge of the peak or drop.

Additionally or alternatively, the processor may be adapted to compute a sum of a total pain amount experienced by the subject over the course of the measurement session and/or a total stress amount experienced over the course of the measurement session. A total pain amount over the measurement session may be computed as a product of a pain level and a time for which the pain was experienced, the pain level being computed for each physiological response event peak or drop having a pain classification, based on one or more properties of the physiological response event peak or drop.

A total non-pain arousal amount over the measurement session may be computed as a product of an arousal level and a time for which the arousal was experienced, the arousal level being computed for each physiological response event peak/drop having a non-pain arousal classification based on one or more properties of the physiological response event peak/drop.

Additionally or alternatively, the processor may be adapted to generate and output, throughout the measurement session, real-time feedback indicative of real-time pain and/or stress experienced by the subject. A user interface may be provided coupled to the processor for generating a sensory output indicative of the feedback.

Optionally, in some embodiments, the processor may further receive a data input indicative of a location in the oral cavity associated with each contact event (i.e. where the contact event took place). This may be linked in data with the physiological response event classification for that contact event. The processor may generate a digital representation of the response classifications as a function of location in the mouth. This may be output to a user interface for display, and/or output to a datastore.

Two example embodiments will now be outlined in more detail.

A first example embodiment is schematically illustrated by Fig. 6 and Fig. 7 and comprises an oral care device for home use by a user, and which integrates system components for performing the pain/arousal classification process described above.

There is provided in accordance with this embodiment an oral care device 72 in the form of a handheld toothbrush having a brush head portion and a handle. The handle houses a motor 74 for driving a drive train mechanism which couples to the brush head and is adapted, when active, to drive the cleaning elements (e.g. bristles) of the brush head to exhibit a cyclical tapping motion 76. In use, the tapping motion is the source of the contact events, i.e. it performs the mechanical engagement with the oral surfaces. The handle further houses a processor 12 which is communicatively coupled with the drive train motor mechanism 74 and is programmed to perform the pain/arousal classification method described in detail in earlier parts of this disclosure. The handle further comprises a set of at least two electrodes 78 coupled to a skin conductance measurement module or circuit (e.g. a galvanic skin response measurement module). A conductive surface of each electrode is exposed at a surface of the handle such that, when a user holds the toothbrush in normal use (for tooth brushing), the electrodes come into contact with the palm of the hand, and the skin conductance measurement circuit generates a skin conductance measurement signal.

Fig. 7 shows an example circuit configuration for the device 72 of Fig. 6. The skin conductance electrodes 78 are connected in series with a resistor, and a current level of the circuit is read and electrically coupled as an input to the processor 12. Thresholding is applied to the skin conductance current via an operational amplifier (comparator), and contingent on the threshold being met (meaning the response is a pain response), a signal is coupled to a communication interface 84 or an I/O. This triggers an adjustment to the brushing mode, wherein a softer brushing mode or setting is selected, so as to reduce the pain of the subject. A data output may also be sent to a mobile device 86 indicative of the detected pain state, and preferably also indicative of the skin conductance signal itself. A computer implemented method may be executed by a processor of the mobile device for summing the total amount of pain experienced over the brushing session.

In some examples, the skin conductance measurement circuit may be electrified only intermittently, on a timing schedule that is controlled in synchrony with the duty cycle of the tapping motion 76 of the brush head. This is illustrated in Fig. 7 which shows the duty cycle timer 82 of the motor 74 coupled to the skin conductance measurement circuit 79, and adapted to electrify the skin conductance circuit simultaneously with each drive action of the motor drive train.

Optionally, there may further be included one or more signal processing components between the skin conductance measurement circuit 79 and the processor 12 for filtering, enhancing and/or modifying the skin conductance signal(s). This may be based on one or more factors such as range of reactivity, a skin conductance baseline measurement, and/or demographic characteristics of the subject.

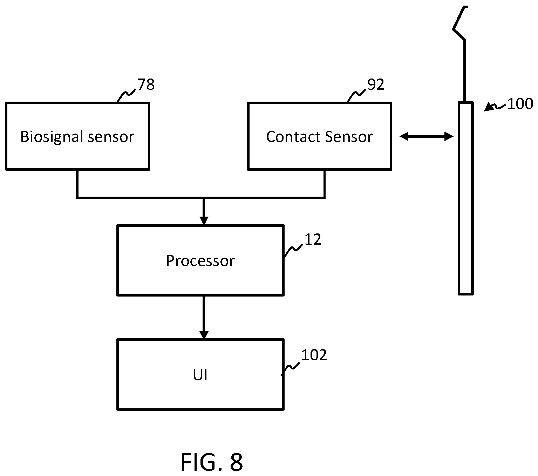

A second example embodiment is schematically represented by Fig. 8 and comprises a system for tracking pain and/or non-pain arousal response of a subject in a clinical environment during a dental procedure or examination. A dental practitioner uses an oral tool 100 such as a dental probe to examine the oral cavity, including applying mechanical stimuli. The mechanical stimuli may comprise pressing or pushing on the teeth, or probing periodontal pockets.

The system comprises a contact sensor 92 for detecting instances of mechanical engagement of the oral tool 100 with a surface within the oral cavity of a subject. There are different options for implementation of the contact sensor. In some examples, the contact sensor may be integrated in the oral tool. The contact sensor may be a force, pressure or stress sensor adapted to detect when the tip of the dental probe is applied against a surface. The contact sensor may be an electrical sensor, e.g. capacitive, resistive, or inductive. The contact sensor may be optical contact sensor. The contact sensor may be a body-channel communication (BCC) sensing means comprising a BCC signal transmitter integrated in the oral tool, adapted to transmit a signal into the body when the oral tool contacts the oral surface, and further comprising a BCC signal receiver coupled to a further part of the patient's body for detecting the signal (or vice versa). The contact sensor may be a magnetic sensor integrated in the oral probe adapted to detect contact with oral tissue via magnetic detection of hemoglobin in blood, using a giant magneto restrictive (GMR) sensor.

The system further includes a biological signal sensing apparatus 78 for measuring a physiological response of the subject. This may for example comprise a skin conductance sensing apparatus such as a Galvanic Skin Response measurement apparatus. This may comprise a set of body-mountable electrodes coupled to a skin conductance measurement module. The electrodes may be coupled to the body for example at the wrist, fingers, palm, or any other location on the body. The electrodes may for example be integrated in a patch, strap or any other body-mountable structure.

The system may further comprise a processor 12 in accordance with any example or embodiment outlined above or described below, or in accordance with any claim of this application. The processor is arranged to receive, over a measurement session, one or more contact signals from the contact sensing means 92 and a biological signal from the biological signal sensing apparatus 78. It is adapted to perform the previously described method for classification of a subject pain and/or non-pain arousal state based on the biological signal and contact signal(s).

The system may further comprise a user interface (UI) 102 to which the classifications may be output for communication to a user. The user interface may comprise a display device for graphical presentation of the feedback. The user interface may comprise an acoustic output device for acoustic feedback, such as an audible sound each time the subject experiences pain, with an acoustic property (e.g. volume or pitch) which changes depending upon pain level. The user interface may comprise one or more light sources, e.g. integrated in the oral tool 100, wherein different illumination patterns of the light sources indicates different pain and/or non-pain arousal states.

Although in the above example, the system comprises a contact sensor to detect each contact event, in alternative examples, a different contact identification means might be provided. For example, a dental practitioner might simply provide an input on a user-input device upon each contact event, and wherein this triggers transmission of a contact signal to the processor. In yet further examples, the indication of the timings of the contact events might be obtained from a set of prompts issued to a user interface prompting a user to apply a certain mechanical stimulus responsive to generation of the prompt (where the prompts may be visual indications on a screen or audible sounds, or haptic signals issued through the tool).

In some examples, the processor 12 may be adapted to perform the classification in real time during the measurement session. The processor 12 may be adapted to generate real-time feedback during the measurement session indicative of the classifications, and to communicate the feedback to a user interface for presentation to a user. Real-time classification feedback enables the dental practitioner to optimize the examination or treatment to minimize pain or negative emotions such as stress.

In some examples, the processor may be adapted to compute the pain level and/or non-pain arousal level associated with each physiological response event, in accordance with the method outlined previously in relation to this. In some examples, the processor may further be adapted to determine a total pain amount and/or a total non-pain arousal amount over the measurement session in accordance with the method outlined previously in relation to this.

In accordance with any of the above described embodiments, the processor 12 may be adapted to acquire a baseline biological signal measurement before any examination or procedure begins. This baseline level may be recorded and used as a reference level for detecting the physiological response events. For example, the criteria for detection of a physiological response event may comprise the biological signal exceeding a pre-determined threshold above the measured baseline.

If the baseline indicates that the subject is under a high level of stress, this may be communicated to the dental practitioner via the UI 102, so that they may seek to calm the subject. During the intervention, they may pause the intervention if stress level rises to a high level relative to the initial baseline. Once the stress level returns to back to the normal baseline value then the dental practitioner may be instructed via the user interface to carry on with the intervention. In case there is no significant decrease in stress level, the dental practitioner may still continue with the procedure. However, reliability of the pain level analysis may be reduced, and a message indicative of this may be indicated to the dental practitioner via the user interface.

A further aspect of the invention provides a computer-implemented method comprising: receiving, during a measurement session, an indication of timings of a series of oral contact events, each contact event corresponding to mechanical engagement of an oral tool with a surface within the oral cavity of a subject; receiving, during the measurement session, a biological signal for the subject from a biological signal sensing apparatus coupled to the subject; identifying physiological response events in the biological signal; determining a temporal proximity between each physiological response event and an immediately preceding contact event, and classifying a subject pain and/or non-pain arousal state associated with the physiological response event based thereon, for example using a classifier algorithm.

Another aspect of the invention provides a computer program product comprising code means configured to cause a processor to perform the method outlined above when the processor is operatively coupled with: a contact identification means adapted for identifying timings of physical engagement of an oral tool with a surface within the oral cavity of a subject; and a biological signal sensing apparatus coupled to the subject.

There are numerous technical advantages achieved by the various embodiments of the present invention.

One advantage is the provision of an objective differential detection of subject pain and non-pain psychological states. A further benefit is that pain and/or non-pain arousal detection can be performed automatically. A further benefit is that pain and/or non-pain arousal detection can be performed at a higher temporal resolution compared to subjective approaches based on verbal feedback from the subject.

A further advantage in accordance with some embodiments is the provision of a quantification of the total pain and/or non-pain arousal experienced over a measurement session. This in turn may be utilized to yield various advantages in different use contexts. For example, when applied in a home-based product, thus may enable remote pain monitoring, which information may be used to inform the scheduling of a next appointment with a dental practitioner. For example, the information about an amount of pain being experienced may be transmitted in a data message to a system accessible by the subject's dental practitioner, and may be used by the dental practitioner to determine a time to schedule a next appointment. This may be based on a current amount of pain being experienced, and/or based on pain trends (e.g. pain is getting worse, or is not improving).

Furthermore, data may optionally be collated from multiple different patients and stored in a datastore. By analyzing the collated data from multiple patients, a dental practitioner may perform critical analysis of their own skills, and spot areas whereby they may improve their skills to reduce pain or psychological stress levels.

Furthermore, a report based on the collated data might be generated which gives average pain levels or amounts for a given dental practitioner for a particular treatment. This could be used to demonstrate practitioner competence, and to reassure patients. This might even decrease subject stress levels before and during an examination or treatment. The information could also be used for marketing purposes.

A further key advantage according to at least one set embodiments is the provision of assessment of the patient's psychological state in real time. This has the consequent advantage that, where applied during an examination or intervention, the dental practitioner can respond directly to the real-time feedback in a feedback loop.

As discussed above, embodiment make use of a processor. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processor adapted for classifying a subject's response to a series of contact events during a measurement session, the processor adapted to:
receive, during the measurement session, an indication of timings of a series of oral contact events, each contact event corresponding to mechanical engagement of an oral tool with a surface within the oral cavity of a subject;
receive, during the measurement session, a biological signal for the subject from a biological signal sensing apparatus coupled to the subject;
identify physiological response events in the biological signal;
determine a temporal proximity between each physiological response event and an immediately preceding contact event, and classify a subject pain and/or non-pain arousal associated with the physiological response event based thereon.

2. A processor as claimed in claim 1, wherein the obtaining the indication of timings comprises receiving one or more contact signals, and optionally wherein the contact signals are received from a contact sensing means adapted for detecting instances of mechanical engagement of the oral tool with an oral surface.

3. A processor as claimed in claim 1 or 2, wherein each contact event has an event occurrence time and an event duration, and wherein the event duration for each contact event is shorter than the time interval between any two successive contact events.

4. A processor as claimed in any of claims 1-3, wherein the biological signal is a skin conductance signal.

5. A processor as claimed in any of claims 1-4, wherein a physiological response event is **characterized by** an event fingerprint in the biological signal comprising a peak or drop in the signal having a peak or drop height exceeding a pre-defined threshold.

6. A processor as claimed in any of claims 1-5, wherein the processor is configured to classify a physiological response event as associated with a pain state dependent on the physiological response event occurring within a pre-defined time window immediately following a contact event.

7. A processor as claimed in claim 6, wherein the classification comprises determining a pain level of the pain state based on one or more properties of the physiological response event peak or drop, and preferably wherein the one or more properties include a maximum height of a leading edge of the peak or drop.

8. A processor as claimed in any of claims 1-7, wherein the processor is configured to classify a physiological response event as associated with a non-pain arousal state dependent on the physiological response event occurring outside of a time window of pre-defined duration following any contact event.

9. A processor as claimed in any of claims 1-8, wherein the classification is performed in real time during the measurement session.

10. A processor as claimed in any of claims 1-9,
wherein the processor is adapted to compute:
a total pain amount over the measurement session, the total pain amount being a product of a pain level and a time for which the pain was experienced, the pain level being computed for each physiological response event peak or drop having a pain classification based on one or more properties of the physiological response event peak or drop; and/or
a total non-pain arousal amount over the measurement session, the total non-pain arousal amount being a product of an arousal level and a time for which the arousal was experienced, the arousal level being computed for each physiological response event peak or drop having a non-pain arousal classification based on one or more properties of the physiological response event peak or drop.

11. A processor as claimed in claim 10, wherein the processor is adapted to trigger one or more response actions dependent on the total pain amount and/or total non-pain arousal amount.

12. A system comprising:
a contact identification means for identifying timings of a series of oral contact events, each contact event corresponding to mechanical engagement of an oral tool with a surface within the oral cavity of a subject;
a biological signal sensing apparatus for measuring a physiological response of the subject; and
a processor as claimed in any of claims 1-11, arranged to receive, over a measurement session, one or more contact signals from the contact identification means indicative of timings of the series of oral contact events and a biological signal from the biological signal sensing apparatus.

13. The system of claim 12, wherein the system includes the oral tool and the contact identification means is a contact sensor integrated in the oral tool, for detecting contact between a portion of the tool and an oral surface.

14. A computer-implemented method comprising:
receiving, during the measurement session, an indication of timings of a series of oral contact events, each contact event corresponding to mechanical engagement of an oral tool with a surface within the oral cavity of a subject;
receiving, during the measurement session, a biological signal for the subject from a biological signal sensing apparatus coupled to the subject;
identifying physiological response events in the biological signal;
determining a temporal proximity between each physiological response event and an immediately preceding contact event, and classifying a subject pain and/or non-pain arousal associated with the physiological response event based thereon.

15. A computer program product comprising code means configured to cause a processor to perform the method of claim 14 when the processor is operatively coupled with
a contact identification means adapted for identifying timings of physical engagement of an oral tool with a surface within the oral cavity of a subject; and
a biological signal sensing apparatus coupled to the subject.
